# EUROPEAN PATENT APPLICATION

(11) **EP 4 322 173 A1**
(43) Date of publication of application: **14.02.2024**
(21) Application number: 22382774.2
(22) Date of filing: 09.08.2022
(51) Int. Cl.: G16H 20/13

(54) **METHODS AND APPARATUS FOR NOTIFYING A USER OF A MEDICAL TREATMENT TO BE ADMINISTERED**

(71) Applicant: Vodafone Group Services Limited, Newbury RG14 2FN (GB); Vodafone España, S.A.U., 28042 Madrid (ES)
(72) Inventor: WORRALL, Chandrika, London, W2 6BY (GB); PUDNEY, Christopher, London, W2 6BY (GB); ROMAGUERA, Cristina, London, W2 6BY (GB)
(74) Representative: Boult Wade Tennant LLP

(57) **Abstract**

Methods and apparatus for notifying a user of a medical treatment to be administered. A control module may determine a medical treatment to be administered by a user, a terminal device registered to the user and a passively powered device associated with the user and associated with an apparatus for administering the medical treatment. A first communication may be transmitted, over a mobile telecommunications network and to the terminal device. The first communication may indicate the medical treatment to be administered. A second communication may be transmitted, over the mobile telecommunications network and to the passively powered device. The second communication may be for causing the passively powered device to output an alert.

## Description

### FIELD OF THE INVENTION

The present disclosure relates to methods, apparatus and software for notifying a user of a medical treatment to be administered. The methods may be implemented in or may otherwise utilise a mobile telecommunications network and a passively powered device for notifying a user of a medical treatment to be administered.

### BACKGROUND

Mobile telecommunications networks, such as cellular networks, are typically capable of providing network connectivity to and communicating with a wide range of different electronic devices. Devices capable of communication over a mobile telecommunications network may include user-operated devices such as mobile telephones (including smartphones), tablets, personal computers and/or Internet of Things (IoT) devices.

A mobile telecommunications network may be used to provide and transmit information related to health care. For example, network connected devices may be used to monitor the health status of one or more users and/or to provide information to a user regarding a health status and/or medical treatment to be administered. Such applications may find particular use in increasingly elderly populations in which a large and increasing proportion of the population is elderly and/or have chronic health conditions.

It is in this context that the subject matter contained in the present application has been devised.

### SUMMARY OF THE INVENTION

It has been realised that improved health outcomes can be delivered by providing fast and efficient ways to notify a user of a medical treatment to be administered. For example, some medical conditions require rapid diagnoses and treatment in order to realise positive health outcomes. It has been realised that such situations may benefit from providing communications to assist a user to locate and identify an apparatus for administering a medical treatment. The methods disclosed herein include the transmission of a communication to a device physically associated with an apparatus for administering a medical treatment. Such a communication may assist a user in locating and identifying the correct apparatus in a short time frame and may lead to earlier treatment and improved health outcomes. In particular, it has been found that a passively powered device capable of receiving a communication transmitted over a mobile telecommunications network and alerting a user to the presence of the passively powered device may be particularly effective at assisting a user in locating an apparatus for administering a medical treatment.

According to a first aspect of the present disclosure there is provided a method of notifying a user of a medical treatment to be administered, the method being implemented in a mobile telecommunications network and comprising: receiving in the mobile telecommunications network: an indication of the medical treatment to be administered, an indication of a terminal device registered to the user and an indication of a passively powered device associated with the user and associated with apparatus for administering the medical treatment, wherein the terminal device and the passively powered device are registered with the mobile telecommunications network; transmitting, over the mobile telecommunications network and to the terminal device, a first communication indicating the medical treatment to be administered; and transmitting, over the mobile telecommunications network and to the passively powered device, a second communication for causing the passively powered device to output an alert.

The first and second communications are transmitted in response to receiving the indication of the medical treatment to be administered, in response to receiving the indication of the terminal registered to the user and in response to receiving the indication of the passively powered device associated with the user and associated with the apparatus for administering the medical treatment.

The first and second communications may be transmitted at substantially the same time.

The passively powered device may comprise an electronic device configured to harvest energy from the environment. For example, the passively powered device may be configured to harvest energy from radio frequency (RF) waves received at the passively powered device. The passively powered device may not include a power source such as a connection to mains electricity or a battery. The passively powered device may include a limited energy storage capability such as one or more capacitors.

The passively powered device may be configured to output an alert in the form of an optical and/or audible alert. For example, the passively powered device may comprise a light source configured to emit visible radiation in response to receiving the second communication. Additionally or alternatively, the passively powered device may comprise a speaker configured to emit a sound in response to receiving the second communication. The passively powered device may be configured to output the alert using energy harvested from the environment.

The apparatus for administering the medical treatment may be any suitable object, equipment, and/or device. In some examples, the medical treatment to be administered may comprise a medicament to be taken. The apparatus for administering the medical treatment may comprise a receptacle for containing the medicament. In some examples, the medical treatment to be administered may comprise treatment with a medical device. The apparatus for administering the medical treatment may comprise a medical device for administering the medical treatment.

The medical treatment may comprise a medical treatment to be administered to the user. Additionally or alternatively, the medical treatment may comprise a medical treatment to be administered by the user and to a patient different to the user.

The method may be implemented in a Radio Access Network (RAN) forming part of the mobile telecommunications network. For example, the method be implemented by one or more base stations forming part of the mobile telecommunications network.

The indication of the medical treatment to be administered, the indication of the terminal device registered to the user and the indication of the passively powered device associated with the user and associated with the apparatus for administering the medical treatment may be received from a control module for determining a medical treatment to be administered by the user.

The method may further comprise receiving, in the mobile telecommunications network, data indicative of a monitored health status and transmitting the data indicative of the monitored health status to the control module.

The control module may be configured to determine a medical treatment to be administered by the user in dependence on the data indicative of a monitored health status. The monitored health status may comprise a monitored health status of the user. Additionally or alternatively, the monitored health status may comprise a monitored health status of a patient to whom the user may administer medical treatment.

The data indicative of a monitored health status may be received from the terminal device or another device registered to the user.

The method may further comprise: receiving in the mobile telecommunications network, an indication that emergency medical treatment is required; and transmitting, over the mobile telecommunications network and to an emergency services provider, an emergency communication indicating that emergency medical treatment is required.

The passively powered device may be associated with and located in proximity to a receptacle containing a medicament to be administered by the user.

The passively powered device may be attached to the receptacle for the medication to be administered by the user.

The method may further comprise: transmitting, over the mobile telecommunications network and to the passively powered device, a third communication for causing the passively powered device to stop outputting the alert.

The method may further comprise: receiving in the mobile telecommunications network, an indication that the passively powered device should stop outputting the alert and transmitting the third communication in dependence on receiving the indication that the passively powered device should stop outputting the alert.

The indication that the passively powered device should stop outputting the alert may be received from a control module for determining a medical treatment to be administered by the user.

The method may further comprise: receiving, in the mobile telecommunications network, an indication that the medical treatment has been administered and transmitting the indication to a control module for determining a medical treatment to be administered by the user.

According to a second aspect of the present disclosure there is provided a method of notifying a user of a medical treatment to be administered, the method comprising: determining, at a control module, a medical treatment to be administered by a user, a terminal device registered to the user and a passively powered device associated with the user and associated with an apparatus for administering the medical treatment, wherein the terminal device and the passively powered device are registered with a mobile telecommunications network; providing to the mobile telecommunications network: an indication of the determined medical treatment to be administered; an indication of the determined terminal device registered to the user; and an indication of the determined passively powered device associated with the user and associated with the apparatus for administering the medical treatment.

The method may further comprise: receiving, at the control module and over the mobile telecommunications network, data indicative of a monitored health status, wherein the determining a medical treatment to be administered to the user comprises determining the medical treatment to be administered to the user in dependence on the received data.

The control module may determine the medical treatment to be administered by the user in dependence on the received data indicative of a monitored health status. The monitored health status may comprise a monitored health status of the user. Additionally or alternatively, the monitored health status may comprise a monitored health status of a patient to whom the user may administer medical treatment.

The data indicative of a monitored health status may be received from the terminal device or another device registered to the user and over the mobile telecommunications network.

The method may further comprise: storing at the control module a record of medical treatments available to the user and passively powered devices associated with apparatus for administering the medical treatments available to the user.

Determining, at the control module, the medical treatment to be administered by the user and the passively powered device may comprise: selecting a medical treatment from the stored record of medical treatments available to the user and selecting the stored passively powered device associated with apparatus for administering the selected medical treatment.

The method may further comprise: storing at the control module, a time schedule for administering the medical treatment by the user. The medical treatment to be administered by the user may be determined in dependence on the stored time schedule.

The method may further comprise: providing to the mobile telecommunications network an indication that the passively powered device should stop outputting an alert.

The method may further comprise: receiving at the control module and over the mobile telecommunications network, an indication that the medical treatment has been administered. The indication that the passively powered device should stop outputting an alert may be provided to the mobile telecommunications network in dependence on receiving the indication that the medical treatment has been administered.

According to a third aspect of the present disclosure there is provided a computer program comprising instructions which, when carried out, cause a method according to any of the first or second aspects to be implemented.

According to a fourth aspect of the present disclosure there is provided a network system in a mobile telecommunications network, the network system being configured to: receive: an indication of a medical treatment to be administered by a user, an indication of a terminal device registered to the user and an indication of a passively powered device associated with the user and associated with apparatus for administering the medical treatment, wherein the terminal device and the passively powered device are registered with the mobile telecommunications network; transmit to the terminal device, a first communication indicating the medical treatment to be administered; and transmit to the passively powered device, a second communication for causing the passively powered device to output an alert.

The network system may comprise a Radio Access Network (RAN). The network system may comprise one or more base stations. The first communication and the second communication may be transmitted by the same or different base stations.

According to a fifth aspect of the present disclosure there is provided a control module in communication with a mobile telecommunications network, the control module being configured to: determine: a medical treatment to be administered by a user; a terminal device registered to the user; and a passively powered device associated with the user and associated with an apparatus for administering the medical treatment, wherein the terminal device and the passively powered device are registered with the mobile telecommunications network; provide to the mobile telecommunications network: an indication of the determined medical treatment to be administered; an indication of the determined terminal device registered to the user; and an indication of the determined passively powered device associated with the user and associated with the apparatus for administering the medical treatment.

In some examples the control module may form part of the mobile telecommunications network. For example, the control module may form part of a Radio Access Network (RAN) (for example, the control module may form part of a base station) and/or a core network of the mobile telecommunications network. In other examples, the control module may be separate from but capable of exchanging communications with the mobile telecommunications network.

The mobile telecommunications network may comprise a fifth generation (5G) New Radio mobile telecommunications network.

Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all examples and/or features of any example can be combined in any way and/or combination, unless such features are incompatible. The applicant reserves the right to change any originally filed claim or file any new claim accordingly, including the right to amend any originally filed claim to depend from and/or incorporate any feature of any other claim although not originally claimed in that manner.

### BRIEF DESCRIPTION OF FIGURES

One or more embodiments of the invention are shown schematically, by way of example only, in the accompanying drawings, in which:
- Figure 1 is a schematic illustration of an example system for notifying a user of a medical treatment to be administered;
- Figure 2 is a schematic illustration of a further example system for notifying a user of a medical treatment to be administered;
- Figure 3 is a flow chart of an example method for notifying a user of a medical treatment to be administered, as implemented at a control module;
- Figure 4 is a flow chart of an example method for notifying a user of a medical treatment to be administered, as implemented in a mobile telecommunications network;
- Figure 5 is a schematic illustration of a still further example system for notifying a user of a medical treatment to be administered; and
- Figure 6 is a schematic illustration of an example electronic device which may be used to implement all or part of any method described herein.

### DETAILED DESCRIPTION

Before particular examples of the present invention are described, it is to be understood that the present disclosure is not limited to the particular examples described herein. It is also to be understood that the terminology used herein is used for describing particular examples only and is not intended to limit the scope of the claims.

In describing and claiming the apparatus and methods of the present invention, the following terminology will be used: the singular forms "a", "an", and "the" include plural forms unless the context clearly dictates otherwise. Thus, for example, reference to "a terminal" includes reference to one or more of such elements.

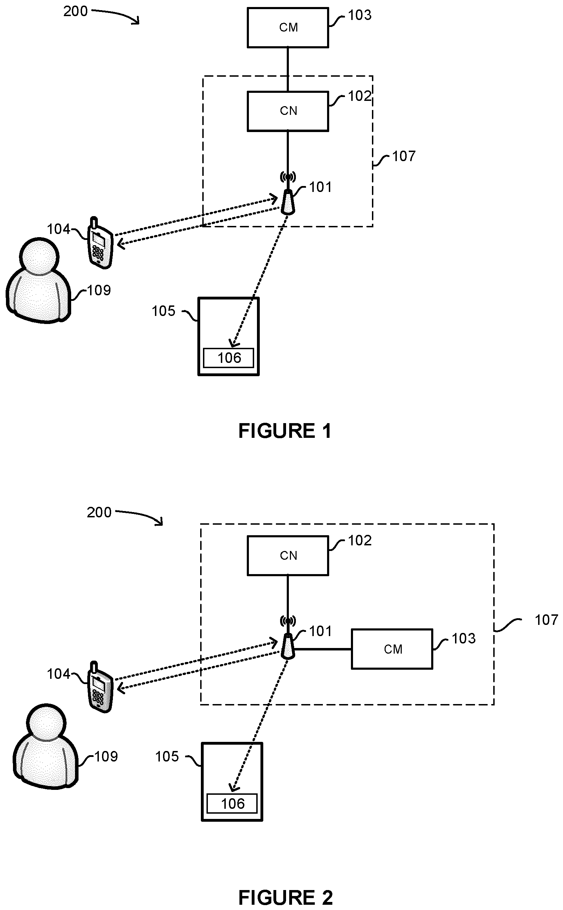

Figures 1 and 2 are schematic illustrations of examples of a system 200 for notifying a user 109 of a medical treatment to be administered. The system 200 includes a terminal device 104, a mobile telecommunications network 107, a control module 103 and a passively powered device 106. The passively powered device 106 is physically associated with (e.g. attached to) an apparatus 105 for administering a medical treatment. The system 200 illustrated in each of Figures 1 and 2 include the same components and differ only by virtue of the arrangement of the control module 103, as will be explained in further detail below.

The apparatus 105 for administering a medical treatment may, for example, comprise a receptacle suitable for containing a medicament. In such examples, the medical treatment to be administered may comprise administering the medicament contained in the receptacle. In other examples, the apparatus 105 for administering a medical treatment may comprise a medical device for administering a treatment. In such examples, the medical treatment to be administered may comprise treatment using the medical device.

The terminal 104 may be any suitable terminal device such as a mobile telephone (e.g. a smartphone), tablet, personal computer, wearable device (e.g. a smartwatch or other form of wearable) etc. The terminal 104 may comprise a user equipment (UE). The terminal 104 is registered with the mobile telecommunications network 107 and is capable of exchanging communications over the mobile telecommunications network 107 over an air interface. The terminal 104 is typically registered with the mobile telecommunications network 107 and to a particular user 109 of the terminal 104.

The mobile telecommunications network includes a radio access network (RAN) and a core network (CN) 102. The RAN typically comprises a plurality of base stations 101 serving at least one cell. For ease of illustration, a single base station 101 is depicted in Figures 1 and 2. The base station 101 comprises at least one antenna configured to exchange communications (e.g. radio frequency signals) with devices (e.g. terminals) situated within a geographical coverage area (which may be referred to as a cell) serviced by the base station 101 over an air interface.

The base station 101 may exchange communications by transmitting and/or receiving communications in one or more frequency bands assigned to a Radio Access Technology (RAT) used by the base station 101 and utilising communication protocols specified for the RAT (e.g. standardised communication protocols for the RAT). Suitable RATs may include, for example, the Global System for Mobile Communications (GSM), the Universal Mobile Telecommunications System (UMTS), Long Term Evolution (LTE) and/or 5G New Radio (NR). The base station 101 may take any suitable form and may, for example, comprise a GSM and/or UMTS compatible base station such as a Node B, an Evolved NodeB (eNB) and/or a 5G NR gNodeB.

The base station 101 is in communication with the core network 102 via a backhaul connection. The core network 102 provides network services to devices (e.g. terminals 104) which are connected to the mobile telecommunications network 107 over the RAN (e.g. via base station 104). For example, the core network 102 may enable the terminal 104 to be connected to the internet via the mobile telecommunications network 107.

The control module 103 comprises one or more electronic devices configured to determine a medical treatment to be administered to a user 109. The control module 103 may take any suitable form such as one or more computing devices which may include one or more of a server, personal computer, tablet, smart phone etc. The control module 103 is capable of exchanging communications with the terminal 104 via the mobile telecommunications network 107. For example, the control module 103 may be connected to the internet and may receive communications from a terminal 104 via its internet connection (which may be provided by the mobile telecommunications network 107). Additionally or alternatively, the control module 103 may send communications to one or more devices connected to the mobile telecommunications network 107 via its internet connection.

In the example arrangement depicted in Figure 1 the control module 103 is shown as being separate to the mobile telecommunications network 107. In the example arrangement depicted in Figure 2 the control module 103 is shown as forming part of the mobile telecommunications network 107. For example, the control module 103 may form part of or be in direct communication with the core network 102 and/or the base station 101 (as illustrated in Figure 2). For the purposes of the examples, disclosed herein, the control module 103 may utilise any suitable connection arrangement for communication with the terminal 104 and passively powered device 106 over the mobile telecommunications network 107.

The passively powered device 106 comprises an electronic device configured to harvest energy from the environment. For example, the passively powered device 106 may be configured to harvest energy from radio frequency waves propagating at the location of the passively powered device 106. Propagating radio frequency waves may originate from a variety of different sources and may, for example, comprise signals transmitted over a mobile telecommunications network, Wireless Fidelity (Wi-Fi) signals, Bluetooth signals etc. Additionally or alternatively, the passively powered device 106 may be configured to harvest energy from radiation (such as optical radiation) received at the passively powered device 106. Typically the passively powered device 106 does not include a substantial in situ power source. For example, the passively powered device 106 may not include a battery and may not be connected (e.g. through a wired or wireless connection) to mains electricity. The passively powered device 106 may include a limited energy storage capability. For example, the passively powered device 106 may include one or more capacitors for storing limited amounts of energy.

For the purposes of the examples described herein, the passively powered device 106 is configured to receive communications transmitted over the mobile telecommunications network 107. For example, the passively powered device 106 may receive communications transmitted from a base station 101 and specifically addressed to the passively powered device 106. The passively powered device 106 may be associated with a unique identifier which allows communications to be specifically addressed to the passively powered device 106. In some examples, the passively powered device may include a radio-frequency identification (RFID) chip or tag. In such examples, the passively powered device 106 may be identified by its unique RFID.

In some examples, a passively powered device 106 may additionally be configured to transmit communications over the mobile telecommunications network 107. Such transmission of communications may comprise backscattering of signals transmitted by a base station 101.

The passively powered device 106 may require and consume relatively low amounts of energy when compared to other forms of electronic device. The passively powered device 106 may additionally or alternatively be relatively inexpensive when compared to other forms of electronic device. The passively powered device 106 may have a low complexity, may be of a relatively small size, may require little or no maintenance and may have a relatively long useful life span.

The passively powered device 106 is configured to output an alert in response to receiving a communication over the mobile telecommunications network 107. For example, the passively powered device 106 may comprise a light source, such as a light emitting diode (LED), configured to emit visible radiation in response to receiving a communication over the mobile telecommunications network 107. Additionally or alternatively, the passively powered device 106 may comprise a speaker configured to emit a sound in response to receiving a communication over the mobile telecommunications network 107. In general, the passively powered device 106 may be configured to output any form of alert which is suitable for alerting the user 109 to the presence of the passively powered device 106 in response to receiving a communication over the mobile telecommunications network 107.

The passively powered device 106 may comprise an Internet of Things (loT) device and may in particular be referred to as a passive or ambient loT device, an ambient power-enabled IoT device and/or an ambient IoT tag.

In the system 200 illustrated in Figures 1 and 2, only a single apparatus 105 and a single associated passively powered device 106 are shown. However, a given user 109 may possess or otherwise have access to a plurality of apparatus 105, each having a respective passively powered device 106, associated with it. For example, a user 109 may possess a plurality of apparatus 105 each for administering a different medical treatment (e.g. a plurality of receptacles containing different medicaments) and each having a respective passively powered device 106 associated with it. That is, a plurality of passively powered devices 106 may be registered or otherwise associated with the same user 109.

In the arrangements depicted in Figures 1 and 2, the mobile telecommunications network 107 is shown as comprising a single base station 101 which serves both the terminal 104 and the passively powered device 106. However, it will be appreciated that generally a mobile telecommunications network 107 comprises a plurality of base stations 101. Depending on their locations, the terminal 104 and the passively powered device 106 may be served by the same or different base stations 101.

The terminal 104 may be registered to a user 109 (e.g. registered with the mobile telecommunications network 107) who may require medical treatment. For example, the terminal 104 may be registered to an elderly person and/or a person having a diagnosed health condition. The apparatus 105 may be for administering a medical treatment suitable for being administered to the user 109 to which the terminal 104 is registered. For example, the apparatus 105 may comprise a receptacle containing medicine which has been prescribed (e.g. by a medical professional) to the user 109. In other examples, the apparatus 105 may comprise a medical device which is suitable for use on the user 109 to which the terminal 104 is registered.

In some examples, the terminal be registered to a user 109 who has care responsibilities for one or more patients (different to the user 109) who may require medical treatment. In such examples, the apparatus 105 may be for administering a medical treatment suitable for being administered to a patient cared for by the user 109 to which the terminal 104 is registered. For example, the apparatus 105 may comprise a receptacle containing medicine which has been prescribed (e.g. by a medical professional) to a patient cared by the user 109. In other examples, the apparatus 105 may comprise a medical device which is suitable for use on a patient cared for by the user 109 to which the terminal 104 is registered. The medical treatment may be administered by the user 109 and to a patient under the user's care.

The control module 103 is configured to determine that a medical treatment should be administered by the user 109 (i.e. the user 109 to which the terminal 104 is registered). The system 200 is configured to notify the user 109 of the medical treatment to be administered. In particular, the system 200 notifies the user 109 of the medical treatment to be administered through a first communication sent to the terminal 104. The system 200 further alerts the user 109 to the apparatus 105 for administering the medical treatment through a second communication sent to the passively powered device 106 which is physically associated with the apparatus 105.

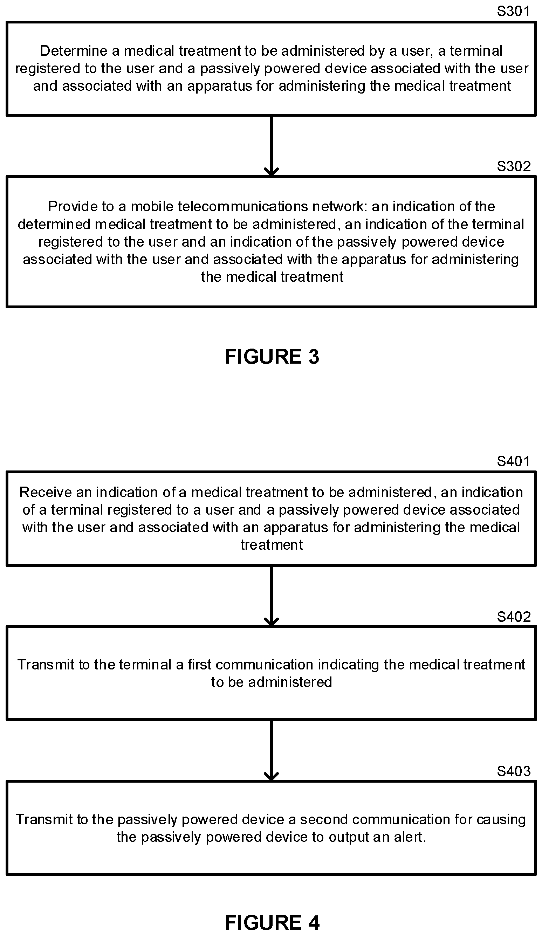

Figure 3 is a flowchart of an example method of notifying a user 109 of a medical treatment to be administered. The method of Figure 3 may be implemented at the control module of the system 200 shown in Figures 1 and 2 and described above.

At step 301, the control module 103 determines a medical treatment to be administered to by the user 109. The control module 103 may determine that a medical treatment should be administered to by the user 109 in dependence on one or more inputs.

In at least some examples, the control module 103 may receive data indicative of a monitored health status. The monitored health status may relate to the health status of the user 109 or a patient under the user's care. The control module 103 may be configured to determine that a medical treatment should be administered by the user 109 in dependence on the received data indicative of a monitored health status. The data indicative of a monitored health status may be received by the control module 103 via the mobile telecommunications network 107. The data indicative of a monitored health status may be received from the terminal 104 registered to the user 109 and/or another device registered to the user 109 (e.g. a wearable device such as a smartwatch). For example, the data indicative of a monitored health status of the user may be collected (e.g. measured) at the terminal 104 and/or other device registered to the user 109. The data indicative of a monitored health status may be collected (e.g. measured) at one or more devices such as a smartphone, wearable device (such as a smartwatch) and/or one or more other sensor devices such as a camera (e.g. positioned in the user's home or in a health or social care environment such as a care home).

The data indicative of a monitored health status may comprise data such as a measured heart rate, temperature, blood pressure, breathing rate, movement status etc. associated with the user 109 or a patient under the user's care. For example, a wearable device (such as a smart watch) worn by the user 109 or a patient may be capable of measuring one or more properties such as the wearer's heart rate, temperature, blood pressure etc. and may send data which is at least indicative of such measured properties to the control module 103 (e.g. over the mobile telecommunications network 107). The data indicative of a monitored health status which is received by the control module 103 may comprise raw measurement data or measured properties and/or may comprise information which is inferred from one or more measured properties. For example, data indicative of a monitored health status, which is received by the control module 103, may comprise a time series of a measured property such as heart rate, temperature, blood pressure etc. and/or may comprise a determined attribute of the measured properties (e.g. an indication that the measured property is abnormal, too high, indicative of a health condition etc.).

The control module 103 may be configured to automatically determine that a medical treatment should be administered by the user 109 in dependence on received data indicative of a monitored health status. For example, the control module 103 may execute one or more software programs and/or algorithms which are configured to determine that a medical treatment should be administered by the user 109 in dependence on received data indicative of a monitored health status. In an example scenario, if a monitored property such as a user's or patient's heart rate diverges from a healthy range (e.g. exceeds or falls below one or more thresholds) then the control module 103 may automatically determine that a medical treatment should be administered by the user 109.

In some examples, the control module 103 may store (e.g. in a memory, which may form part of the control module 103) a time schedule for administering a medical treatment by the user 109. The time schedule may, for example, be prescribed by a medical professional and may comprise times and/or a frequency at which a medical treatment should be administered. The control module 103 may determine that a medical treatment should be administered according to the time schedule. For example, the control module 103 may determine that a medical treatment should be administered when a current time (e.g. a time according to a clock implemented by the control module 103) reaches a time which is designated for administering a medical treatment in the stored time schedule.

In at least some examples, the control module 103 may be capable of receiving user input. For example, the control module 103 may implement a user interface for receiving user input. Additionally or alternatively the control module 103 may be in communication with an electronic device implementing a user interface for receiving user input. For example, the control module 103 may be in network communication (e.g. via an internet connection and/or via a mobile telecommunications network 107) with an electronic device implementing a user interface and may receive from the electronic device user inputs receiving at the electronic device. The user inputs may be provided by a medical professional. For example, the control module 103 may administer a user account registered to a medical professional. The user account may provide access, through the control module 103 (e.g. via a user interface), to data related to the medical status of the user 109 (or a patient under the user's care) to which a medical treatment is to be administered. The data may, for example, comprise received data indicative of a monitored health status and/or a time schedule for administering a medical treatment by the user 109 as were described above. The data may be viewed by a medical professional by providing login credentials (or any other suitable form of user accreditation) at a suitable device implementing a user interface.

The medical professional may provide a user input indicating a medical treatment to be administered by the user 109. For example, a medical professional may deduce from data related to the medical status of the user 109 (or a patient under the user's care) that a medical treatment should be administered by the user 109 and may provide a suitable user input which is received by the control module 103. In response to the user input the control module 103 may, in some examples, update a stored time schedule for administering a medical treatment by the user 109. Additionally or alternatively, the control module 103 may determine that a medical treatment should be administered by the user 109 in direct dependence on the user input provided by a medical professional.

The control module 103 may store (e.g. in a memory, which may form part of the control module 103) a record of medical treatments available to the user 109. For example, any medical treatments which have been prescribed, provided and/or registered to the user 109 (or a patient under the user's care) may be stored in a record of medical treatments available to the user. The control module 103 may further store a record of a passively powered device 106 which is associated with apparatus for administering each medical treatment which is available to the user. For example, for each available medical treatment a record may be stored of a passively powered device 106 which is physically associated with (e.g. attached to) an apparatus for administering the medical treatment. The record may, for example, include sufficient information to address a communication to the passively powered device 106. Such a record may, for example, comprise at least one identifier of a passively powered device 106. Suitable identifiers may include identifiers such as an international mobile equipment identity (IMEI), an international mobile subscriber identity (IMSI), and/or a subscription permanent identifier (SUPI). In some examples, the passively powered device 106 may comprise a radio-frequency identification (RFID) chip or tag. In such examples, the passively powered device 106 may be identified by its unique RFID. The record stored at the control module 103 may include an RFID of the passively powered device 106.

As was explained above, a given user 109 may have a plurality of passively powered devices 106 registered to them or otherwise associated with them. Each passively powered device 106 may be associated with a different apparatus 105 for administering a medical treatment. The control module 103 may store a record of a plurality of medical treatments available to a user along with information (e.g. an identifier) indicating a passively powered device associated with apparatus for administering each medical treatment.

Determining a medical treatment to be administered to a user at step 301 of the method of Figure 3 may comprise selecting, at the control module 103, a medical treatment from the stored record of medical treatments available to the user 109.

Also at step 301 the control module 103 further determines a passively powered device 106 associated with apparatus 105 for administering the determined medical treatment. As was explained above, the control module 103 may store a record of medical treatments available to a user 109 along with information indicating passively powered devices associated with apparatus for administering the medical treatments. For example, the record may include identifiers (such as an IMEI, IMSI, RFID and/or SUPI) registered to passively powered devices 106 associated with apparatus 105 for administering available medical treatments. Determining a passively powered device 106 associated with apparatus 105 for administering the determined medical treatment may comprise selecting the passively powered device 106 stored in the record and associated with apparatus for administering the determined medical treatment. Determining a passively powered device 106 may comprise selecting an identifier (e.g. an IMEI, IMSI, RFID and/or SUPI) associated with the selected passively powered device 106.

Also at step 301 the control module 103 further determines a terminal device 104 registered to the user 109. The control module 103 may store a record of at least one terminal device 104 registered to the user 109. For example, the control module 103 may store a mobile telephone number or other form of identifier such as an IMEI, IMSI and/or SUPI associated with at least one terminal 104 registered to the user. Determining a terminal device 104 registered to the user 109 may comprise selecting the terminal device 104 which is registered to the user 109 (by whom the medical treatment is to be administered) from the stored record of terminal devices 104. Determining a terminal device 104 may comprise selecting an identifier (e.g. telephone number, an IMEI, IMSI and/or SUPI) associated with the terminal device 104.

At step 302 of the method of Figure 3 the control module 103 provides to the mobile telecommunications network 107 an indication of the determined medical treatment to be administered. That is, the control module 103 provides an indication of the medical treatment which was determined in step 301. The indication of the medical treatment to be administered may be configured for providing to a terminal device 104. The indication of the medical treatment to be administered may comprise a message to the user 109 instructing them to administer the determined medical treatment. The message may comprise text to be displayed to or otherwise communicated to (e.g. by outputting sound corresponding to a reading of the message) the user 109. The indication may take any suitable form such as a Short Message Service (SMS) message, a Multimedia Messaging Service (MMS) message, an instant messaging service message, a message configured for a specific application executed by a terminal device 104 and/or an email message.

The indication of the medical treatment to be administered may be provided as a push notification or a pull notification. For example, the control module 103 may push the indication to the mobile telecommunications network 107 without a specific request from the terminal 104 for retrieving the indication. Alternatively, the control module 103 may provide the indication to the mobile telecommunications network 107 in response to a request (a pull request) from the terminal 104 to retrieve any indications of a medical treatment to be administered. In such examples, the determining of the terminal device 104 in step 301 may comprise determining the terminal device 104 which has made a pull request to the control module 103.

Also at step 302 of the method of Figure 3, the control module 103 provides, to the mobile telecommunications network 107, an indication of the determined terminal device 104 registered to the user 109. That is, the control module 103 provides an indication of the terminal device 104 which was determined in step 301. The indication of the determined terminal device 104 may take any form suitable for identifying the terminal device 104 to which the indication of the medical treatment to be administered is to be provided. The indication of the terminal device 104 may allow the mobile telecommunications network 107 to route the indication of the medical treatment to be administered to the terminal 104. For example, the indication may comprise an identifier of the terminal device 104 such as a telephone number, an IMEI, IMSI and/or SUPI. The indication of the determined terminal device 104 may be included in a first communication addressed to the terminal 104 and including the indication of the medical treatment to be administered.

Also at step 302 of the method of Figure 3, the control module 103 provides to the mobile telecommunications network 107 an indication of the determined passively powered device 106 associated with the user 109 and associated with the apparatus 105 for administering the determined medical treatment. That is, the control module 103 provides an indication of the passively powered device 106 which was determined in step 301. The indication of the determined passively powered device 106 may take any form suitable for identifying the passively powered device 106. The indication of the passively powered device 106 may allow the mobile telecommunications network 107 to route a communication to the passively powered device 106. For example, the indication may comprise an identifier of the passively powered device 106 such as an IMEI, IMSI, RFID and/or SUPI. The indication of the determined passively powered device 106 may be included in a second communication addressed to the passively powered device 106 and for causing the passively powered device 106 to output an alert.

Figure 4 is a flowchart of an example method of notifying a user 109 of a medical treatment to be administered. The method of Figure 4 may be implemented in the mobile telecommunications network 107 shown in Figures 1 and 2 and described above. The method may be implemented in a network system forming part of the mobile telecommunications network 107. For example, the method may be implemented in a Radio Access Network (RAN) forming part of the mobile telecommunications network 107. The method may be implemented in one or more base stations 101 forming part of the mobile telecommunications network 107.

At step 401 an indication of a medical treatment to be administered is received in the mobile telecommunications network 107. The indication received in the mobile telecommunications network 107 at step 401 may correspond with the indication of the medical treatment to be administered which is provided by the control module 103 in step 302 of the method of Figure 3 described above. Any of the features or properties described above with reference to the indication of a medical treatment to be administered in step 302 of the method of Figure 3 may also apply to the indication received in the mobile telecommunications network 107 at step 401 of the method of Figure 4. For example, the indication may comprise a message to the user 109 to be provided to a terminal 104 instructing them to administer the determined medical treatment. The indication may, for example, be in the form of a message to be transmitted by the mobile telecommunications network 107 to the terminal 104.

Also at step 401 of the method of Figure 4, an indication is received of a terminal 104 registered to the user 109 by whom the medical treatment is to be administered. The terminal 104 represents a terminal 104 to which the indication of the medical treatment to be administered is to be sent. The indication of the terminal 104 received in the mobile telecommunications network 107 at step 401 may correspond with the indication of the terminal 104 which is provided by the control module 103 in step 302 of the method of Figure 3 described above. Any of the features or properties described above with reference to the indication of a terminal 104 in step 302 of the method of Figure 3 may also apply to the indication received in the mobile telecommunications network 107 at step 401 of the method of Figure 4. For example, the indication may comprise an identifier of the terminal 104 such as a telephone number, an IMEI, IMSI and/or SUPI. The received indication of the terminal 104 may be packaged with the indication of the medical treatment to be administered, for example, in the form of a message addressed to the terminal 104 (the contents of the message including the indication of the medical treatment to be administered).

Also at step 401 of the method of Figure 4, an indication is received of a passively powered device associated with a user 109 (by whom the medical treatment is to be administered) and an apparatus 105 for administering the medical treatment. The passively powered device 106 represents a device which is associated with (e.g. physically associated with, such as being attached to) apparatus for administering the medical treatment which is indicated by the control module 103. The indication of the passively powered device 106 received in the mobile telecommunications network 107 at step 401 may correspond with the indication of the passively powered device 106 which is provided by the control module 103 in step 302 of the method of Figure 3 described above. Any of the features or properties described above with reference to the indication of a passively powered device 106 in step 302 of the method of Figure 3 may also apply to the indication received in the mobile telecommunications network 107 at step 401 of the method of Figure 4. For example, the indication may comprise an identifier of the passively powered device 106 such as an IMEI, IMSI, RFID and/or SUPI. The indication of the passively powered device 106 may, for example, be received in the form of a communication addressed to the passively powered device 106.

At step 402 of the method of Figure 4, a first communication indicating the medical treatment to be administered is transmitted, over the mobile telecommunications network 107, and to the terminal device 104. The terminal device 104 to which the first communication is transmitted is the terminal device 104 for which an indication is received from the control module 103 in step 401. The indication of the medical treatment to be administered is the indication which is received from the control module 103 in step 401. The first communication may be routed through the mobile telecommunications network 107 and transmitted from a base station 101 which serves the terminal device 104 to which the first communication is addressed. The first communication may take any suitable form and may, for example, comprise one or more of an SMS message, an MMS message, an instant messaging service message, a message configured for a specific application executed by a terminal device 104 and/or an email message.

In response to receiving the first communication, the terminal 104 may display a notification or otherwise alert a user 109 of the terminal 104 to the received communication. The terminal 104 may further display or otherwise output (e.g. by outputting sound corresponding to the indication of the medical treatment to be administered) the indication of the medical treatment to be administered. For example, the terminal 104 may display a message informing the user 109 that a medical treatment should be administered and further informing the user 109 of the type of medical treatment to be administered. In general, the first communication transmitted to the terminal 104 may serve to alert a user 109 of the terminal of the medical treatment to be administered.

At step 403 of the method of Figure 4, a second communication is transmitted, over the mobile telecommunications network 107, and to the passively powered device 106. The passively powered device 106 to which the second communication is transmitted is the passively powered device 106 for which an indication is received from the control module 103 in step 401. The second communication may be routed through the mobile telecommunications network 107 and transmitted from a base station 101 which serves the passively powered device 106 to which the second communication is addressed.

The second communication is for causing the passively powered device to output an alert. As was explained above, the passively powered device 106 is configured to output an alert in response to receiving a communication over the mobile telecommunications network 107. For example, the passively powered device 106 may comprise a light source, such as an LED, configured to emit visible radiation in response to receiving the second communication over the mobile telecommunications network 107. Additionally or alternatively, the passively powered device 106 may comprise a speaker configured to emit a sound in response to receiving the second communication over the mobile telecommunications network 107. In general, the passively powered device 106 may be configured to output any form of alert which is suitable for alerting the user 109 to the presence of the passively powered device 106 in response to receiving the second communication over the mobile telecommunications network 107.

In the flowchart of Figure 4 the first communication is shown as being transmitted to the terminal 104 before the second communication is transmitted to the passively powered device 106. However, the time order of steps 402 and 403 may be reversed, or steps 402 and 403 may be implemented at substantially the same time. For example, the second communication may be transmitted to the passively powered device 106 before the first communication is transmitted to the terminal 104. Alternatively, the first communication may be transmitted to the terminal 104 and the second communication may be transmitted to the passively powered device 106 at substantially the same time. In general, it may be advantageous for the first and second communication to be transmitted at substantially the same time or at least with a relatively short time gap between the transmissions. This will allow the user to be alerted to the medical treatment to be administered via the terminal 104 and to the apparatus 105 for administering the medical treatment at the same or similar times.

The passively powered device 106 to which the second communication is addressed is physically associated with an apparatus 105 for administering the medical treatment (i.e. the medical treatment determined by the control module 103 at step 301 of the method of Figure 3). That is, the passively powered device 106 is physically associated with an apparatus 105 for administering the medical treatment which the user 109 is instructed to administer in the indication sent to the terminal 104 in the first communication (step 402 of Figure 4).

For example, the medical treatment to be administered may comprise taking of a first medicament. The indication of the medical treatment which is sent to the terminal 104 in the first communication may comprise a message instructing the user 109 to take the first medicament (or instructing the user 109 to administer the first medicament to a patient under their care). The passively powered device 106 may be physically associated with (e.g. attached to) a receptacle containing the first medicament. In such an example, the first communication serves to inform the user 109 that a medical treatment should be administered and the second communication serves to assist the user 109 in locating the apparatus 105 for administering the medical treatment.

The user 109 may posses a plurality of apparatus for administering medical treatments (e.g. a plurality of receptacles containing medication). The second communication and the subsequent alert output by the passively powered device 106 associated with the apparatus 105 for administering the medical treatment (e.g. the receptacle containing the required medicament) will allow a user to quickly locate and identify the correct apparatus 105 for administering the medical treatment from the plurality of apparatus in their possession. For example, the user may possess a plurality of medicament receptacles located in a cupboard. The alert output by the passively powered device 106 associated with the correct medicament receptacle will allow the user to quickly locate the receptacle containing the correct medicament in the cupboard. This may significantly reduce the amount of time before the medical treatment is successfully administered, thereby improving the health outcome of the person to which the medical treatment is administered.

Whilst a particular example was described above in which the medical treatment to be administered comprises a medicament to be taken by the user 109 (or a patient under the user's care), in other examples the medical treatment may take other forms. For example, the medical treatment to be administered may comprise the use of a medical device. In such examples, the passively powered device 106 may be physically associated (e.g. attached to) to a medical device for administering the medical treatment. Similarly to the above described example, the alert output by the passively powered device 106 may assist a user 109 to quickly locate and identify the correct medical device (e.g. from a plurality of medical devices) for administering the medical treatment. This may significantly reduce the amount of time before the medical treatment is successfully administered, thereby improving the health outcome of the person to which the medical treatment is administered.

As was explained above, whilst a single apparatus 105 and a single passively powered device 106 is shown in Figures 1 and 2, a system 200 for notifying a user 109 of a medical treatment to be administered may include a plurality of apparatus 105 each having an associated passively powered device 106. Passively powered devices 106 are advantageously low energy devices (i.e. they require relatively low amounts of energy to function) and may be relatively low cost when compared to other forms of electronic device. These or other characteristics of passively powered device 106 make them suitable for associating with (e.g. attaching to) several different apparatus 105 for administering medical treatments and facilitating quick and easy location of a correct apparatus 105 for administering a medical treatment.

As was explained above, the passively powered device 106 is capable of receiving a second communication transmitted over a mobile telecommunications network 107. Advantageously, this allows the passively powered device 106 to receive a communication and output an alert over a wide geographical region. For example, a user may take one or more apparatus 105 for administering a medical treatment with them when leaving their home. Whilst outside of a home and within a coverage area of the mobile telecommunications network 107, passively powered devices 106 associated with the one or more apparatus 105 may still receive communications over the mobile telecommunications network 107. This enables quick location and identification of a correct apparatus 105 for administering a medical treatment in any location served by the mobile telecommunications network 107.

In an example scenario, a user 109 may leave their home and partake in exercise. The user 109 may carry with them (e.g. in a bag) at least one apparatus 105 for administering a medical treatment. The user 109 may, for example, wear a wearable device which monitors the user's heart rate. The exercise which is taken by the user 109 may result in the user's heart rate exceeding a threshold. The user's measured heart rate and/or an indication that the heart rate has exceeded a threshold may be communicated to the control module 103 (e.g. over the mobile telecommunications network 107) in the form of data indicative of a monitored health status of the user 109. The control module 103 may determine (e.g. in dependence on the received data indicative of a monitored health status of the user 109) that medical treatment should be administered by the user 109. For example, the control module 103 may determine that the user should take a medicament in response to the user's heart rate exceeding a threshold.

In accordance with the methods described above with reference to Figures 3 and 4, a first communication may be transmitted to a terminal 104 registered to the user 109 and the second communication may be issued to a passively powered device 106 associated with an apparatus 105 for administering the medical treatment (e.g. a receptacle containing the required medicament). The user 109 may view a notification displayed on their terminal 104 in response to receiving the first communication and may thereby be informed that they should administer the determined medical treatment. The user 109 may then attempt to locate an apparatus 105 for administering the medical treatment (e.g. by looking in their bag). In response to receiving the second communication, a passively powered device 106 associated with the apparatus 105 for administering the required medical treatment may output an alert. The alert output by the passively powered device 106 will assist the user 109 in locating and identifying the apparatus 105 and may allow the medical treatment to be administered quickly, thereby improving health outcomes for the user 109. For example, the medical treatment may quickly reduce the risk to the user posed by their increased heart rate.

In at least some examples, a third communication may be transmitted to the passively powered device 106 for causing the passively powered device 106 to stop outputting the alert. For example, the third communication may be for causing the passively powered device 106 to turn off a light source and/or to stop outputting a sound. The third communication may be transmitted over the mobile telecommunications network 107 in a similar way to the transmission of the second communication.

The third communication may be transmitted in response to an indication that the medical treatment has been administered. For example, a user 109 may provide an input (e.g. using a suitable user interface) at the terminal 104 confirming that the medical treatment indicated in the first communication (and received at the terminal 104) has been administered. In response to an input received at the terminal 104, the terminal 104 may transmit a communication to the control module 103 (e.g. over the mobile telecommunications network) indicating that the medical treatment has been administered. The control module 103 may, in response to receiving the indication from the terminal 104, provide an indication to the mobile telecommunications network 107 that the passively powered device 106 should stop outputting an alert. The indication may, for example, comprise a communication addressed to the passively powered device 106. The mobile telecommunications network 107 may then transmit the third communication to the passively powered device 106 for causing the passively powered device 106 to stop outputting the alert.

As was explained above, in some examples a third communication may be transmitted to the passively powered device 106 for causing the passively powered device 106 to stop outputting the alert. In other examples, the passively powered device 106 may stop outputting the alert after a pre-configured period of time has expired. For example, upon receipt of the second communication, the passively powered device 106 may output an alert and may start a timer. Upon expiry of the timer, the passively powered device 106 may stop outputting the alert and without further communication over the mobile telecommunications network 107.

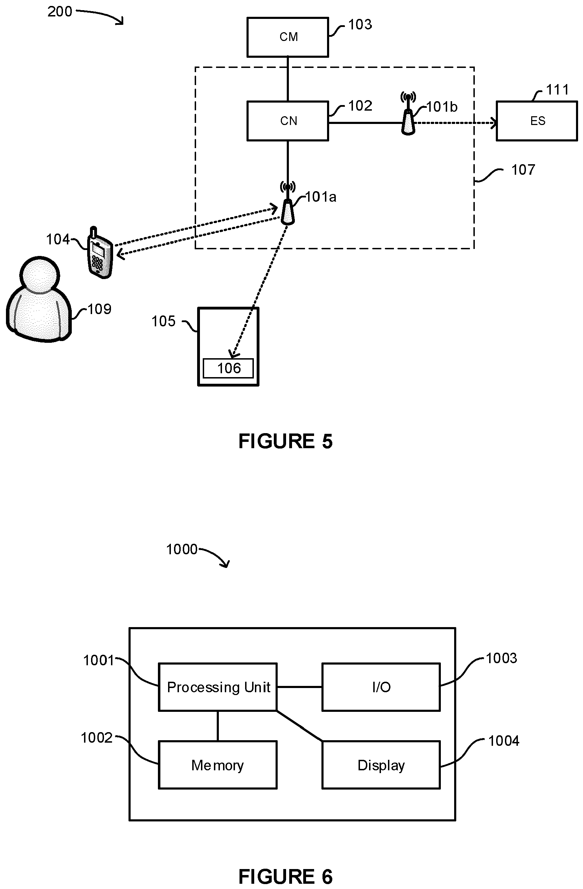

Figure 5 is a schematic illustration of a further example of a system 200 for notifying a user 109 of a medical treatment to be administered. The system 200 shown in Figure 5 includes many of the same or corresponding components to the systems shown in Figures 1 and 2 and described in detail above. Any of the features or properties described above with reference to the systems 200 shown in Figures 1 and 2 may also apply to corresponding components of the system 200 shown in Figure 5. No detailed description of the same or corresponding features will therefore be provided with reference to Figure 5.

The system 200 shown in Figure 5 differs relative to the systems 200 of Figures 1 and 2 by virtue of including an emergency service (ES) provider 111. The emergency service provider 111 may be registered with or may otherwise be contactable by the mobile telecommunications network 107. In the example shown in Figure 5, the ES provider 111 is depicted as being served by a second base station 101b. The terminal 104 and passively powered device 106 are shown as being served by a first base station 101a. However, any of the terminal 104, passively powered device 106 and ES provider 111 may be served by a single base station 101 or any suitable combination of a plurality of base stations 101. In the example, shown in Figure 5, the control module 103 is shown as being separate to and in communication with the mobile telecommunications network 107. However, in other examples, the control module 103 may form part of the mobile telecommunications network as was described above with reference to Figure 2.

The system 200 shown in Figure 5 may transmit an emergency communication (e.g. via the mobile telecommunications network 107) to the ES provider 111 indicating that emergency medical treatment is required. For example, the control module 103 may determine that emergency medical treatment is required. Such a determination may be made, for example, in dependence on data indicative of a monitored health status received at the control module 103. For example, the control module 103 may determine that the received data indicative of a monitored health status indicates that the user or patient to whom the data relates requires emergency medical treatment. Additionally or alternatively, a medical professional may provide an input to the control module 103 indicating that emergency medical treatment is required.

The control module 103 may provide an indication to the mobile telecommunications network 107 that emergency medical treatment is required. The indication may, for example, comprise a message addressed to the ES provider 111 and providing information regarding the required emergency treatment (e.g. identifying the person requiring treatment and their location). The mobile telecommunication network 107 may transmit an emergency communication to the ES provider 111 indicating that emergency medical treatment is required. The emergency communication may, for example, comprise the message addressed to the ES provider 111.

In addition to the emergency communication transmitted to the ES provider 111, the system 200 of Figure 5 may additionally transmit a first communication to the terminal 104 and a second communication to the passively powered device 106 as was described in detail above. This may facilitate the administration of medical treatment prior to the arrival of the emergency services.

Several examples have been described above in the context of administering medical treatment in a patient's home. However, the methods and devices described herein are not limited to use in a patient's home and may have application in a variety of different settings. For example, the methods and devices described herein may find application in health and/or social care settings. For example, the methods and devices described herein may be used in a setting such as a care home, a doctor's surgery, a hospital and/or an operating theatre.

Several examples have been described herein in the context of notifying a user of a medical treatment to be administered to the same user which is notified of the medical treatment. For example, a user may be notified of a medical treatment to administer on themselves. However, in other examples the user who is notified of the medical treatment (the user 109 in the examples described above) may not be the person to receive the medical treatment. For example, the user may be notified of a medical treatment to administer to a patient under the notified user's care.

In some examples, in the systems depicted in Figures 1, 2 and 5 and described above, the user 109 with which the terminal 104 is registered may be a user 109 with care responsibilities for one or more patients. Using the methods and devices described herein the user 109 may be notified of a medical treatment to be administered by the user 109 and to a patient under the user's care. In such examples, the first and second communication transmitted to the terminal 104 and the passively powered device 106 respectively will notify the user 109 of the medical treatment to administer to a patient and assist the user in locating and identifying an apparatus 105 for administering the medical treatment.

Various methods have been described herein which may be implemented on any suitable electronic device (such as a computing device) and/or combination of electronic devices (e.g. computing devices). Figure 6 is a schematic illustration of an example electronic device which may be used to implement all or part of any method described herein. The general structure of the device depicted in Figure 6 may be applicable to any terminal 104, base station 101, network node (such as component of a core network 102), control module 103, passively powered device 106 and/or any other electronic device contemplated herein.

The device 1000 may include at least one processing unit 1001, memory 1002 and an input/output (I/O) interface 1000. The processing unit 1001 may include any suitable processer and/or combination of processors. For example, the processing unit 1001 may include one or more of a Central Processing Unit (CPU) and a Graphical Processing Unit (GPU). The memory 1002 may include volatile memory and/or non-volatile/persistent memory. The memory 1002 may, for example, be used to store data such as an operating system, instructions to be executed by the processing unit (e.g. in the form of software to be executed by the processing unit), configuration information related to the device 1000, session information and/or configuration or registration information associated with any other device, node or module in the network. In some examples, the memory 1002 may be used to store instructions for executing any of the methods disclosed herein.

At least the processing unit 1001 is connected to an input/output (I/O) interface 1003. The I/O interface 100 facilitates communication with one or more other devices, network nodes or modules in a network. For example, the I/O interface 1003 may be operable to transmit and/or receive communications to/from other devices in a network. In some examples, the I/O interface 1003 may be operable to transmit and/or receive communications over an air interface. For example, the I/O interface 1003 may include a transmitter and/or a receiver for transmitting and/or receiving wireless communication (e.g. radio frequency signals). In some examples, the I/O interface 1003 may include a transceiver configured to receive and transmit wireless communication (e.g. radio frequency signals). In some examples, the I/O interface 1003 may be operable to additionally or alternatively communicate over one or more wired connections.

Optionally, the device 1000 may further include a display 1004. For example, where the device 1000 is a terminal 104, the terminal may include a display 1004 for displaying information to a user 109 of the terminal. The display 1004 may comprise any suitable electronic display such as a touch sensitive display. The display 1004 may be connected to at least to the processing unit 1001. The processing unit 1001 may generate display signals which are sent to the display 1004 in order to cause the display information.

In the interest of conciseness not all possible alternatives which fall within the scope of the present disclosure have been explicitly discussed herein. As the skilled person will appreciate, in the present disclosure any aspect discussed from the perspective of an element being operable to do an action also discloses the same feature from the perspective of a method including a method step corresponding to the action. Similarly, any discussion presented from the perspective of a method step also discloses the same features from the perspective of any one or more suitable elements being operable or configured to carry out some or all of the method step. It is also considered within the present disclosure that for any method step(s), there can be a computer program configured to carry out, when executed, the method step(s).

Within the context of the present disclosure a device, such as a terminal, a base station, or a network module or node, or server is generally considered from a logical perspective, as the element carrying out the appropriate function. Any such device may be implemented using one or more physical elements as deemed appropriate. For example, it may be implemented in one (or more) of: a standalone physical device, in two or more separate physical devices, in a distributed system, in a virtual environment using any suitable hardware or hardware combination, etc.

It will be appreciated that embodiments of the present invention can be realised in the form of hardware, software or a combination of hardware and software. Any such software may be stored in the form of volatile or non-volatile storage such as, for example, a storage device like a ROM, whether erasable or rewritable or not, or in the form of memory such as, for example, RAM, memory chips, device or integrated circuits or on an optically or magnetically readable medium such as, for example, a CD, DVD, magnetic disk or magnetic tape. It will be appreciated that the storage devices and storage media are embodiments of machine-readable storage that are suitable for storing a program or programs that, when executed, implement examples of the present disclosure. Accordingly, examples provide a program comprising code for implementing a system or method as claimed in any preceding claim and a machine readable storage storing such a program. Still further, examples of the present disclosure may be conveyed electronically via any medium such as a communication signal carried over a wired or wireless connection and examples suitably encompass the same.

Features, integers, characteristics, or groups described in conjunction with a particular aspect, embodiment or example of the invention or disclosure are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing examples.

## Claims

1. A method of notifying a user of a medical treatment to be administered, the method being implemented in a mobile telecommunications network and comprising:
receiving in the mobile telecommunications network: an indication of the medical treatment to be administered, an indication of a terminal device registered to the user and an indication of a passively powered device associated with the user and associated with apparatus for administering the medical treatment, wherein the terminal device and the passively powered device are registered with the mobile telecommunications network;
transmitting, over the mobile telecommunications network and to the terminal device, a first communication indicating the medical treatment to be administered; and
transmitting, over the mobile telecommunications network and to the passively powered device, a second communication for causing the passively powered device to output an alert.

2. A method according to claim 1, wherein the indication of the medical treatment to be administered, the indication of the terminal device registered to the user and the indication of the passively powered device associated with the user and associated with the apparatus for administering the medical treatment are received from a control module for determining a medical treatment to be administered by the user.

3. A method according to claim 2, further comprising: receiving, in the mobile telecommunications network, data indicative of a monitored health status and transmitting the data indicative of the monitored health status to the control module,
wherein optionally the data indicative of a monitored health status is received from the terminal device or another device registered to the user.

4. A method according to any preceding claim, further comprising:
receiving in the mobile telecommunications network, an indication that emergency medical treatment is required; and
transmitting, over the mobile telecommunications network and to an emergency services provider, an emergency communication indicating that emergency medical treatment is required.

5. A method according to any preceding claim, wherein the passively powered device is associated with and located in proximity to a receptacle containing a medicament to be administered by the user,
wherein optionally the passively powered device is attached to the receptacle for the medication to be administered by the user

6. A method according to any preceding claim, further comprising:
transmitting, over the mobile telecommunications network and to the passively powered device, a third communication for causing the passively powered device to stop outputting the alert,
wherein optionally the method further comprises:
receiving in the mobile telecommunications network, an indication that the passively powered device should stop outputting the alert and transmitting the third communication in dependence on receiving the indication that the passively powered device should stop outputting the alert.

7. A method according to any preceding claim, further comprising:
receiving, in the mobile telecommunications network, an indication that the medical treatment has been administered and transmitting the indication to a control module for determining a medical treatment to be administered by the user.

8. A method of notifying a user of a medical treatment to be administered, the method comprising:
determining, at a control module, a medical treatment to be administered by a user, a terminal device registered to the user and a passively powered device associated with the user and associated with an apparatus for administering the medical treatment, wherein the terminal device and the passively powered device are registered with a mobile telecommunications network;
providing to the mobile telecommunications network:
an indication of the determined medical treatment to be administered;
an indication of the determined terminal device registered to the user; and
an indication of the determined passively powered device associated with the user and associated with the apparatus for administering the medical treatment.

9. A method according to claim 8, further comprising: receiving, at the control module and over the mobile telecommunications network, data indicative of a monitored health status,
wherein the determining a medical treatment to be administered to the user comprises determining the medical treatment to be administered to the user in dependence on the received data,
wherein optionally the data indicative of a monitored health status is received from the terminal device or another device registered to the user and over the mobile telecommunications network.

10. A method according to claim 8 or 9, further comprising: storing at the control module a record of medical treatments available to the user and passively powered devices associated with apparatus for administering the medical treatments available to the user,
wherein optionally determining, at the control module, the medical treatment to be administered by the user and the passively powered device comprises: selecting a medical treatment from the stored record of medical treatments available to the user and selecting the stored passively powered device associated with apparatus for administering the selected medical treatment.

11. A method according to any of claims 8-10, further comprising: storing at the control module, a time schedule for administering the medical treatment by the user,
wherein the medical treatment to be administered by the user is determined in dependence on the stored time schedule.

12. A method according to any of claims 8-11, further comprising: providing to the mobile telecommunications network an indication that the passively powered device should stop outputting an alert,
wherein optionally the method further comprises: receiving at the control module and over the mobile telecommunications network, an indication that the medical treatment has been administered, wherein the indication that the passively powered device should stop outputting an alert is provided to the mobile telecommunications network in dependence on receiving the indication that the medical treatment has been administered.

13. A computer program comprising instructions which, when carried out, cause a method according to any of claims 1-12 to be implemented.

14. A network system in a mobile telecommunications network, the network system being configured to:
receive: an indication of a medical treatment to be administered by a user, an indication of a terminal device registered to the user and an indication of a passively powered device associated with the user and associated with apparatus for administering the medical treatment, wherein the terminal device and the passively powered device are registered with the mobile telecommunications network;
transmit to the terminal device, a first communication indicating the medical treatment to be administered; and
transmit to the passively powered device, a second communication for causing the passively powered device to output an alert.

15. A control module in communication with a mobile telecommunications network, the control module being configured to:
determine: a medical treatment to be administered by a user; a terminal device registered to the user; and a passively powered device associated with the user and associated with an apparatus for administering the medical treatment, wherein the terminal device and the passively powered device are registered with the mobile telecommunications network;
provide to the mobile telecommunications network:
an indication of the determined medical treatment to be administered;
an indication of the determined terminal device registered to the user; and
an indication of the determined passively powered device associated with the user and associated with the apparatus for administering the medical treatment.
